# EUROPEAN PATENT APPLICATION

(11) **EP 4 588 424 A1**
(43) Date of publication of application: **23.07.2025**
(21) Application number: 25150606.9
(22) Date of filing: 08.01.2025
(51) Int. Cl.: A61B 1/303, A61B 1/31, A61B 1/32, A61B 1/015

(54) **DEVICE FOR INSPECTING A CAVITY**

(30) Priority: 16.01.2024 IT 202400000678
(71) Applicant: THD S.p.A., 42015 Correggio, Reggio Emilia (IT)
(72) Inventor: BASTIA, FILIPPO, 41019 SOLIERA (IT)
(74) Representative: Paparo, Aldo

(57) **Abstract**

Device (1) for inspecting a cavity, comprising:
a dilator body (2), having a prevalent extension along a longitudinal axis (X) between a proximal end (21) and a distal end (22), wherein the proximal end (21) and the distal end (22) are connected by a side wall (23) and define, together with said side wall (23), an inner volume (V); wherein
the proximal end (21) identifies a proximal opening (PO), and is connected to a gripping element (24), said proximal element (21) being couplable with a detecting device; wherein the distal end (22) identifies a distal opening (DO), for access to the tissues delimiting the orifice from the inner volume (V); and wherein the side wall (23) comprises at least one side opening (LO) connecting the inner volume (V) with the outside environment.
an introducing body (3), having a gripping portion (31), accessible or graspable by the operator, connected to an insertion portion (32); wherein said insertion portion (32) has elongated extension along the longitudinal axis X between an insertion end (33), of substantially ogival shape, distal with respect to the gripping portion (31), and an intermediate portion (34) proximal with respect to the gripping portion (31); said insertion portion (32) being shaped to be introduced into the inner volume (V) through the side opening (LO) of the dilator body (2), following a transverse direction (Y); and said insertion portion (32) being shaped to be moved inside the inner volume (V) parallel to the longitudinal axis (X) between a stroke start position, in which the insertion end (33) is proximal to the side window (LO), and a stroke end position, in which the insertion end (33) protrudes from the distal opening (DO) of the dilator body (2).

## Description

The present invention relates to a device for inspecting a cavity.

The present invention is advantageously applied in the sector of diagnosis of pathologies relating to human, natural or surgically obtained cavities or orifices, a diagnosis that is performed by means of visual inspection of the tissues delimiting said cavities and said orifices.

In particular, but not exclusively, the present invention is advantageously applied in the sector of anorectal and vaginal inspection: consequently, the object of the present invention has preferably, but not exclusively, the alternative embodiments of anoscope, proctoscope, rectoscope and vaginal speculum.

Inspection devices comprising a dilator body and an introducing body are known in the prior art: the dilator body is hollow and has a tubular extension between a distal opening to access visually and operatively the mucous membranes delimiting the cavity, and a proximal opening, accessible by the operator at the moment of use; the introducing body also has a tubular extension, between a distal end of substantially ogival shape and a proximal end, graspable by the operator.

The introducing body is configured to be coupled to the dilator body by sliding of said introducing body inside the dilator body, until the ogival end of the introducing body protrudes from the distal end of the dilator body, obtaining outer walls shaped to facilitate insertion of the inspection device into the cavity of the patient.

During an inspection examination, after said coupling between the dilator body and the introducing body, the operator inserts the inspection device into the cavity of the patient, and then extracts the introducing body from the dilator body, maintaining said dilator body inserted inside the cavity and allowing visual access and, therefore, inspection of the mucous membrane through the distal opening of the dilator body.

The dilator bodies in the prior art can be coupled in various ways to detecting instruments, such as cameras, configured for the detection of images from the inspected cavity: said coupling is obtained by the operator once the dilator body is correctly positioned in the cavity of the patient.

Patent application number EP3329834, in the name of the same Applicant, illustrates a proctoscope comprising said dilator body and said introducing body. The introducing body, in detail, is configured to be inserted through the proximal opening of said dilator body, until the configuration of complete insertion is achieved, with the ogival distal end protruding from the distal opening of the dilator body.

Furthermore, the dilator body has, at the proximal opening, coupling means configured to couple an adaptor body, suitable for connecting a detecting device, for example the aforesaid camera for detection of images of the anorectal cavity: the camera, once positioned, is substantially coaxial with the extension axis of the tubular body.

The patent application also illustrates a method for inspecting the anal cavity, comprising the steps of: inserting the dilator body coupled to the introducing body, extracting the introducing body, by removing it through the proximal opening of the dilator body, coupling the adaptor body to the coupling means located at the proximal opening and, lastly, connecting the detecting device to the adaptor body.

However, the proctoscope illustrated and, consequently, the steps described above, could certainly be improved.

In particular, the proctoscope illustrated can be improved in terms of assembly of the inspection device, facilitating said assembly for the operator; furthermore, said proctoscope can be improved, making the step of coupling the proctoscope with the detecting device more comfortable for the patient.

The technical task of the present invention is to provide a device for inspecting a cavity that allows the limits of the prior art to be overcome.

In particular, an object of the present invention is to provide a device for inspecting a cavity that is easier to use for the operator and that improves the comfort for the patient, once it has been inserted into the cavity.

A further object of the present invention is to provide a device for inspecting a cavity that is versatile and easy to use.

The technical task set and the objects specified are substantially achieved by a device for inspecting a cavity comprising the technical features as set out in one or more of the accompanying claims.

The dependent claims correspond to possible embodiments of the invention.

Further features and advantages of the present invention will become more apparent from the following indicative and therefore non-limiting, description
of a preferred but not exclusive embodiment of a device for inspecting a cavity, as illustrated in the accompanying drawings, in which:
- figure 1 shows a side view of a device for inspecting a cavity according to the present invention, in a disassembled configuration,
- figure 2 shows a rear isometric view of the inspection device of figure 1;
- figure 3 shows an isometric view from below of the device for inspecting a cavity according to the present invention, in an assembled configuration;
- figure 4 shows a side view of the inspection device of figure 3.

An object of the present invention is a device 1 for inspecting a cavity by an operator. Said device 1 is preferably an anoscope or a rectoscope, for inspecting an anal or rectal cavity of a patient. Alternatively, said device 1 is a vaginal speculum, for inspecting the vaginal cavity of a patient. However, devices suitable for inspecting different cavities, natural or surgically obtained in the patient, are not excluded.

The terms "distal" and "proximal" will be used in the description. Such terms are, in this context, to be intended as with respect to the operator: the term "proximal" is to be intended as referring to the parts furthest away from the patient, whereas the term "distal" is to be intended as referring to the parts closest to the patient.

The device 1 according to the present invention comprises a dilator body 2 and an introducing body 3.

The dilator body 2 is shaped to be inserted into the cavity of the patient on whom it is intended to perform the inspection. Said dilator body has an elongated extension along a longitudinal axis X, between a proximal end 21 and a distal end 22, connected by a side wall 23; the side wall 23 is, therefore, substantially tubular; in a non-exclusive embodiment, the side wall 23 extends in a diverging fashion from the distal end 22 to the proximal end 21, to facilitate dilation of the cavity to be explored.

The side wall 23 delimits and identifies, together with the proximal end 21 and the distal end 22, an inner volume V.

The proximal end of the dilator body identifies a proximal opening PO accessible by the operator and crossed by the longitudinal axis X; in a non-exclusive embodiment, the proximal opening PO is coaxial with the longitudinal axis X; similarly, the distal end 22 identifies a distal opening DO, preferably coaxial with the longitudinal axis X and configured to allow access from the inner volume V to the tissues and the mucous membranes delimiting the cavity.

The proximal opening PO and the distal opening DO have a substantially circular shape.

Furthermore, the proximal end 21 can be coupled with a detecting device, for example a camera, for detecting images from the explored cavity. In detail, the proximal end 21 comprises one or more hooking sites, configured to reversibly constrain said detecting device: positioned at the proximal opening PO and constrained to the hooking site, the detecting device is turned towards the inner volume V and can operate, detecting images, film clips or other parameters of interest to the operator.

The proximal end 21 is also connected to a gripping element 24, accessible by the operator and, directly or indirectly, graspable by the operator. Preferably, the gripping element 24 is a handle, tilted by an angle comprised between 0 and 90° with respect to the longitudinal axis X, in a vertical direction.

The gripping element 24 can also comprise one or more hooking sites 26, for coupling with diagnostic and surgical instruments or, in any case, useful for completion of the inspection examination; for example, the gripping element 24 can comprise a hooking site 26 for an aspirator of air or other substances from the inner volume V.

The side wall 23 has at least one side opening LO connecting the inner volume V with the outside environment: the side opening LO is preferably obtained in a proximal portion of the dilator body 2.

The device 1 according to the present invention further comprises an introducing body 3, having a gripping portion 31 and an insertion portion 32.

The gripping portion 31 is situated proximal to the insertion portion 32 and is accessible to or graspable by the operator: in detail, the gripping portion 31 is shaped to allow the operator to grasp and manoeuvre said introducing body 3 easily.

The insertion portion 32, connected to the gripping portion, has an elongated extension along the longitudinal axis X between an insertion end 33, of substantially ogival shape, which is distal from the gripping portion 31, and an intermediate portion 34 that is proximal with respect to the gripping portion 31.

The insertion portion 32 is configured to be inserted into the inner volume V identified by the dilator body 2, to obtain an assembled configuration of the device 1, which can be easily inserted into the cavity to be explored.

In other words, the introducing body 3 is reversibly couplable to the dilator body 2 for introduction of the insertion portion 32 into the inner volume V, to obtain the assembled configuration of the device 1.

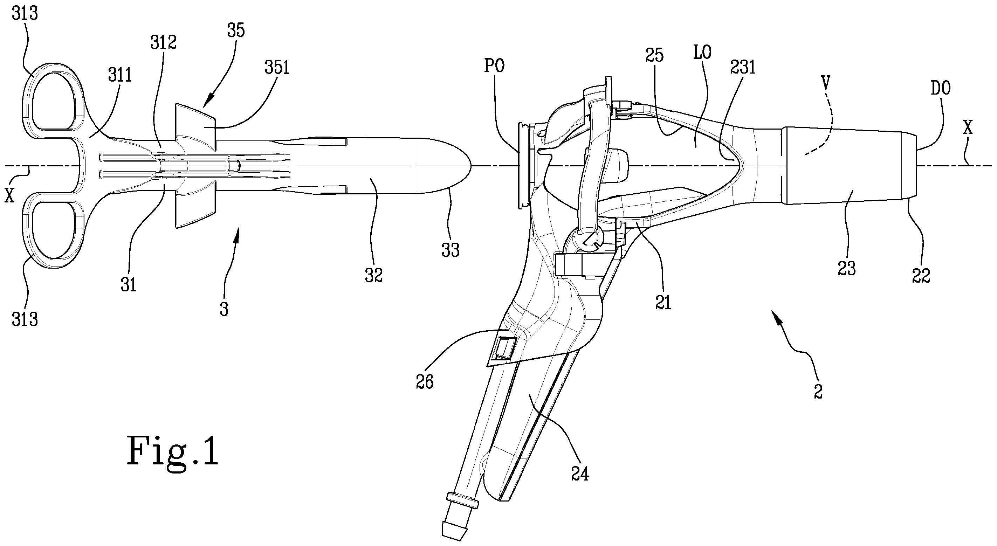

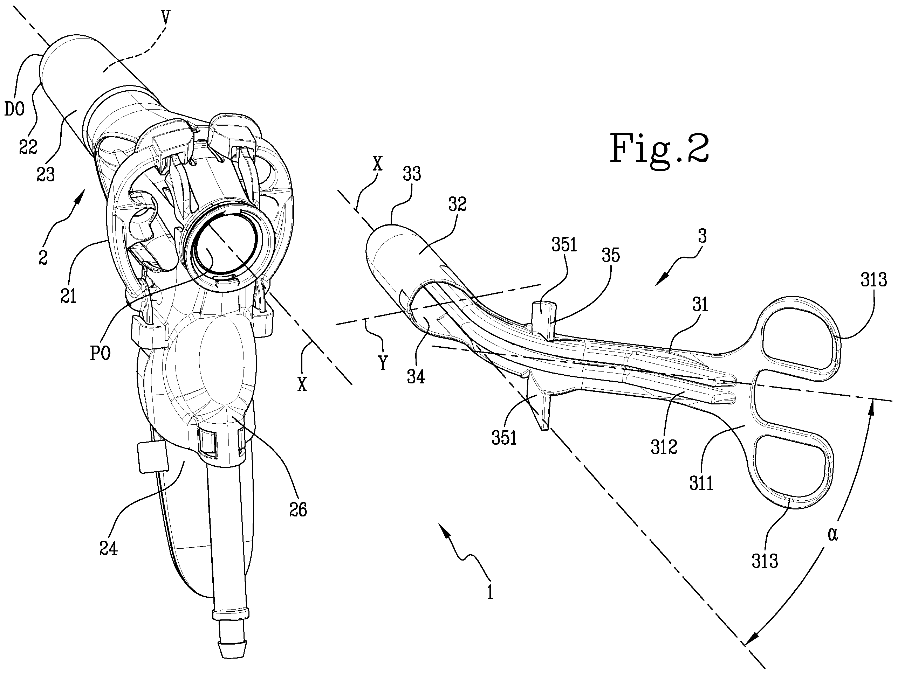

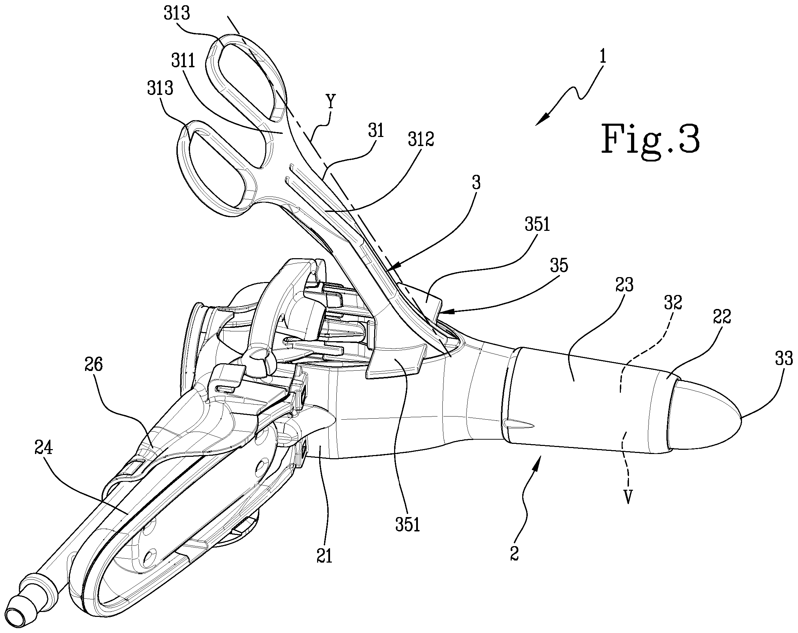

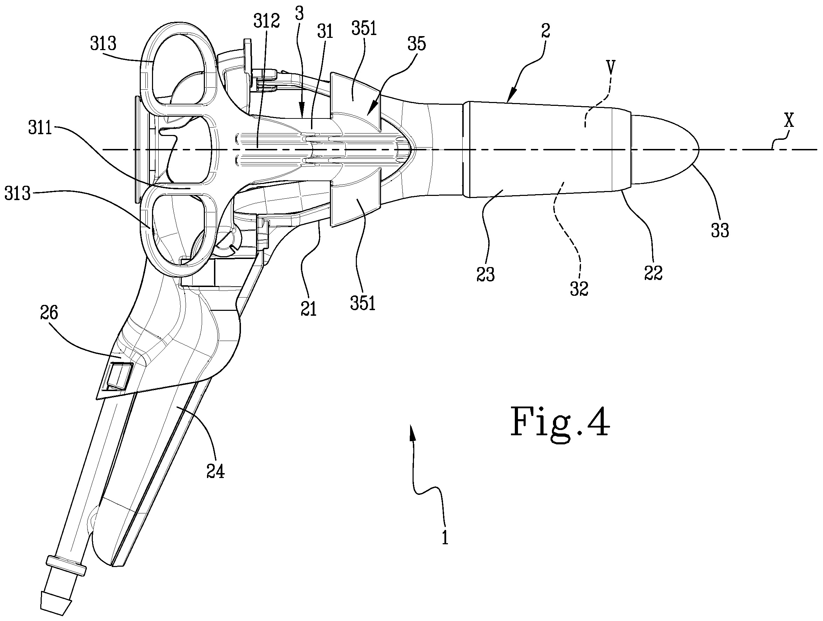

Figures 1 and 2 show, in detail, a device 1 comprising a dilator body 2 and an introducing body 3, in a disassembled configuration. However, in order to allow easy insertion of the dilator body 2 inside the cavity to be explored, the dilator body 2 and the introducing body 3 are assembled, to form said device 1. Figures 3 and 4 show the device 1 in the assembled configuration, with the introducing body 3 inserted into the dilator body 2.

In detail, the assembled configuration is obtained by insertion of the introducing body 3 into the dilator body 2 following an insertion trajectory: firstly, the insertion portion 32 is introduced into the inner volume V of the dilator body 2 through the side opening LO, following a transverse direction Y. Subsequently, the insertion portion 32 is moved parallel to the longitudinal axis X, between a stroke start position, in which the insertion end 33 is proximal to the side window LO, and a stroke end position, in which the insertion end 33 protrudes from the distal opening DO of the dilator body 2.

In detail, the insertion trajectory of the introducing body 3 into the dilator body 2 comprises a first section having an extension in the transverse direction Y between a first position, in which the introducing body 3 and, in particular, the insertion portion 32, is situated externally to the dilator body 2, and a second position, in which the introducing body 3 and, in particular, the insertion end 33 is inserted into the inner volume V identified by the dilator body 2.

Passing from the first to the second position, i.e. travelling the first section of the insertion trajectory, the dilator body and, in particular, the insertion end 33, crosses the side window LO obtained from the side wall 23. Therefore, when the second position is reached, the insertion portion 32 is at least partially inserted inside the dilator body 3.

The introducing body 3, when moved by the operator along the first section of the insertion trajectory, passes from a disengaged configuration to a configuration of being at least partially inserted inside the inner volume V of the dilator body 2.

At the end of the first section of the insertion trajectory, the insertion end 33 is located in proximity to the side opening LO.

The insertion trajectory further comprises a second section, having a substantially parallel extension to the longitudinal axis X between the stroke start position, in which the insertion portion 32 is at least partially located inside the dilator body 2 and the insertion end 33 is proximal to the side opening LO, and the stroke end position, in which the insertion end 33 protrudes from the dilator body 2, through the distal opening DO.

In other words, the stroke end position is reached by the introducing body 3 by advancement of the insertion portion 32 from the stroke start position to the stroke end position.

In order to obtain the assembled configuration of the device 1, the insertion portion 32 is counter-shaped with respect to the inner volume V delimited by the side wall 23.

For example, a substantially cylindrical insertion portion 32 corresponds to a substantially cylindrical inner volume V.

Alternatively, a similarly tapered insertion portion 32 corresponds to a tapered inner volume V.

Movement of the insertion portion 32 between the stroke start position and the stroke end position occurs preferably by sliding of said insertion portion 32 inside the dilator body 2; in particular, once an inner surface 231 delimiting internally the side wall 23 of the dilator body is defined, the insertion portion is sliding along said inner surface 231 or part of it.

For example, but not exclusively, the inner surface 231 may have one or more sliding guides: the insertion portion 32, couplable with said sliding guides, is configured to slide along the sliding guides between the stroke start position and the stroke end position.

In other words, when the introducing body 3 is inserted into the dilator body 2, the insertion portion 32 reaches the stroke end position, with the insertion end 33, of ogival shape, protruding from the distal opening DO of the dilator body: the device 1 in assembled configuration therefore has an elongated extension, diverging from the insertion end 33 of the introducing body 2 to the proximal end 21 of the dilator body 2; the device 1 also does not have steps or discontinuous elements externally that could make insertion and positioning of the device 1 in the cavity of the patient unpleasant or painful.

In detail, in fact, the fact that the insertion portion 32 is counter-shaped with respect to the volume V guarantees that the device 1, in the assembled configuration, does not have the aforesaid steps or discontinuous elements externally. Externally, therefore, the device 1 has a substantially ogival and uniform profile, to guarantee easy insertion into the cavity of the patient.

Furthermore, the device 1 according to the present invention may comprise locking elements for locking the introducing body 3, said locking elements reversibly constraining the introducing body 3 to the dilator body 2, when the insertion portion 32 of the dilator body 3 reaches the stroke end position: the locking elements, of the type known in the art, allow the introducing body to be maintained constrained to the dilator body 2 during insertion of the device 1; in other words, thanks to the constraining elements, the device 1 can be advantageously inserted by the operator using a single hand, without running the risk of the introducing body 3, upon insertion of the device 1 into the cavity, being moved with respect to the dilator body 2 in the opposite direction to the insertion one, as a result of the resistance forces exerted, for example, by the muscle structures surrounding the cavity of the patient.

Once the device 1 is inserted by the operator into the cavity of the patient, the introducing body 3 is removed from the dilator body 2, following an extraction trajectory, opposite with respect to the insertion trajectory: firstly, the insertion portion 32 of said introducing body 3 is moved parallel to the longitudinal axis X from the stroke end position to the stroke start position situated in proximity to the longitudinal opening LO; then, the insertion portion 32 is moved in the transverse direction Y through the side window LO, determining the extraction of the insertion portion 32 from the inner volume V and, consequently, removal of the introducing body 3 from the dilator body 2.

Advantageously, the device 1 according to the present invention, comprising said dilator body 2 having the side window LO and said introducing body 3, allows the operator to assemble and disassemble the device 1 rapidly, intuitively and easily, following respectively the aforesaid insertion trajectory and the aforesaid extraction trajectory.

Advantageously, the device 1 according to the present invention allows assembly and disassembly of the device 1 without having to remove any hooking sites and/or any detecting devices coupled to the dilator body 2 at the proximal opening PO before each assembly and disassembly operation and, therefore, before each insertion of the device 1 into the cavity and before each extraction of the introducing body 3 from the dilator body 2.

Advantageously, therefore, the operations of assembly of the device 1, of insertion of the device 1 into the cavity and of removal of the introducing body 3 from the dilator body 2 are more rapid and efficient for the operator, and have an improved level of comfort for the patient.

Inside the device 1, the dilator body 2 and the introducing body 3 can assume different configurations, provided these allow said assembly following the insertion trajectory having a first extension in the transverse direction Y and a second extension along the longitudinal axis X.

In particular, the side opening LO and the insertion portion 32 can assume different configurations.

Firstly, the side opening LO can be situated to the right or to the left of the proximal opening PO, considering the gripping element 24 positioned vertically and downwards; furthermore, the side opening LO can be delimited by an edge 25 and have a substantially oval shape or, in any case, elongated along the longitudinal axis X.

In the preferred embodiment of the device 1 according to the present invention, illustrated in figures 1-4, the dilator body 2 has two elongated side openings LO, obtained from the side wall 23, arranged symmetrically with respect to the longitudinal axis X and laterally with respect to the gripping element 24.

However, other configurations of the dilator body 2 and, in particular, of the side opening LO, are not excluded.

Similarly, the introducing body 3 can also have the gripping portion 31 and the insertion portion 32 of different three-dimensional conformations.

In a first embodiment of the introducing body 3, the gripping portion 31 comprises a gripping end 311 shaped to facilitate gripping and movement of the introducing body 3 and a connecting wall 312, interposed between the gripping end 311 and the insertion portion 32. Preferably, the connecting wall 312 is tilted by an angle α with respect to the longitudinal axis X, said angle α being comprised between 0° and 90°.

In the preferred embodiment, illustrated in figures 1-4, the introducing body 3 has a gripping portion 31 having two rings 313, shaped for insertion of the operator's fingers. Advantageously, the gripping portion 31 having two rings 313 facilitates both the operations of insertion of the introducing body 3 into the dilator body 2 following the insertion trajectory, and operations of extraction of the insertion body 3 from the dilator body 2 following the extraction trajectory.

Advantageously, therefore, the device 1 in the preferred embodiment of figures 1-4 is particularly easy to handle, both in the disassembled configuration and the assembled configuration and in the passage from assembled configuration to disassembled configuration and vice versa.

Furthermore, the introducing body 3 can comprise at least one stop element 35 configured to signal to the operator that the stroke end position has been reached; said stop element 35 can assume different configurations and can be located in different sites along the introducing body 3. Preferably, the stop element 35 is located at the intermediate portion 34.

In the preferred embodiment of the device 1 illustrated in figures 1-4, the introducing body 3 has a stop element 35 comprising an abutment surface 351, extending on a substantially vertical plan parallel to the longitudinal axis X; said abutment surface 315 is configured to enter into contact with a portion of the edge 25 of the side window LO, when the insertion body 3 reaches the stroke end position; in other words, the abutment surface 351, by entering into contact with the edge 25, prevents the introducing body 3 from being further inserted into the dilator body 2.

Other configurations of the abutment surface 351 or the stop element 35, easily deducible by the person skilled in the art, are also not excluded.

Lastly, in an alternative embodiment, the introducing body 3 has, at the intermediate portion 34, a viewing opening, coaxial with the longitudinal axis X and configured to allow a view of the mucous membranes and tissues delimiting the cavity, even when the introducing body 3 is inserted into the dilator body 2. Preferably, therefore, the dilator body and the introducing body are both made of transparent polymeric material, in order to allow said view of the tissues and surrounding mucous membranes.

In a further embodiment, the introducing body 3 can comprise a magnifying device at the intermediate portion 34 or at the insertion end 33, in order to allow a magnified view of the tissues and the mucous membranes delimiting the cavity; advantageously, thanks to the presence of the magnifying device, the operator is able to view a greater number of details, improving the quality of the inspection examination.

In conclusion, the device 1 in the variants described is quicker and easier to assemble/disassemble with respect to the inspection devices of the prior art, as well as being manageable and easy to use for the operator.

As stated in previous paragraphs, the inspection device 1 according to the present invention can advantageously be used to facilitate the introduction into and inspection of a cavity. A method of use of said device 1 can comprise the following steps:
- Preparing a dilator body 2 of the type described in the preceding paragraphs and having hooking sites for a detecting device.
- Preparing an introducing body 3 of the type described in the preceding paragraphs;
- Assembling the device 1, by introducing the introducing body 3 into the dilator body 2, following the insertion trajectory described above;
- Introducing the device 1 at least partially into the cavity of the patient;
- Extracting the introducing body 3 from the dilator body 2, following the extraction trajectory described above;
- Performing the inspection of the cavity to be explored;

Furthermore, the method can comprise the steps of:
- Coupling a detecting device to the hooking sites at the dilator body 1, before or after introduction of the device 1 into the cavity of the patient; and
- Detecting images or other parameters of interest relating to the cavity to be explored.

The method described allows removal to be avoided of any hooking sites or other adaptor bodies for detecting devices located at the proximal opening PO of the dilator body 2, each time it is intended to insert the introducing body 3 into the dilator body 2 and each time it is intended to extract the introducing body 3 from the dilator body 2.

Said hooking sites and possible said detecting devices can be advantageously maintained coupled to the dilator body 2 for the entire duration of the inspection examination, from assembly of the device 1 and during extraction of the introducing body 3 from the dilator body 2.

Advantageously, in conclusion, the device 1 simplifies the assembly operations and makes them quicker and, overall, makes insertion of the device 1 into the cavity more comfortable for the patient and easier for the operator.

## Claims

1. A device (1) for inspecting a cavity, comprising:
a dilator body (2), extending prevalently along a longitudinal axis (X), between a proximal end (21) and a distal end (22), wherein the proximal end (21) and the distal end (22) are connected by a side wall (23) and define, together with said side wall (23), an inner volume (V);
wherein the proximal end (21) identifies a proximal opening (PO) crossed by said longitudinal axis (X) and is connected to a gripping element (24), which is accessible or graspable by an operator, said proximal end (21) being couplable with a detecting device;
wherein the distal end (22) identifies a distal opening (DO), for accessing the tissues delimiting the orifice from the inner volume (V); and
wherein the side wall (23) comprises at least one side wall (LO) connecting the inner volume (V) with the outside environment.
said device (1) further comprising:
an introducing body (3), having a gripping portion (31), which is accessible or graspable by the operator, connected to an insertion portion (32);
wherein said insertion portion (32) extends along the longitudinal axis X between an insertion end (33), of substantially ogival shape, which is distal from the gripping portion (31), and an intermediate portion (34) that is proximal with respect to the gripping portion (31),
said inspection device (1) being **characterized in that**:
said insertion portion (32) is shaped to be introduced into the inner volume (V) through the side opening (LO) of the dilator body (2), following a transverse direction (Y); and
said insertion portion (32) being shaped to be moved inside the inner volume (V) parallel to the longitudinal axis (X) between a stroke start position, in which the insertion end (33) is proximal to the side window (LO) and a stroke end position, in which the insertion end (33) protrudes from the distal opening (DO) of the dilator body (2).

2. The device (1) according to claim 1 comprising locking elements for locking the introducing body (3), said locking elements reversibly constraining the introducing body (3) on the dilator body (2) when the insertion portion (32) of the dilator body (3) reaches the stroke end position.

3. The device (1) according to claim 1 wherein the side wall (23) of the dilator body is delimited inside by an inner surface (231) having a sliding guide, and wherein the insertion portion (32) of the introducing body (3) is slidable along said sliding guide until the stroke end position is reached.

4. The device (1) according to claim 1 wherein the introducing body (3) comprises a stop element (35), signalling to the operator that the stroke end position has been reached.

5. The device (1) according to claim 4 wherein the stop element (35) is located at the intermediate portion (34).

6. The device (1) according to claim 4 or 5 wherein the opening (LO) of the dilator body is delimited by an edge (25) and wherein the stop element (35) comprises at least one abutment surface (351) configured to come into contact with a portion of said edge (25) when the stroke end position is reached, preventing the introducing body (3) from being inserted further into the dilator body (2).

7. The device (1) according to any one of the preceding claims wherein the intermediate portion (34) defines a viewing opening that is coaxial with respect to the longitudinal axis (X).

8. The device according to claim 7 wherein the introducing body (3) comprises a magnifying device located at the intermediate portion (34) or the insertion end (33), permitting a magnified view of the tissues delimiting the cavity.

9. The device according to one of the preceding claims wherein the gripping portion (31) comprises a gripping end (311) connected to a connecting wall (312),
said gripping end (311) facilitating gripping and moving the introducing body (3); and
said connecting wall (312) being interposed between the gripping end (311) and the insertion portion (32) and being tilted by an angle (α) relative to the longitudinal axis (X).

10. The device according to claim 9 wherein the angle (α) is comprised between 0 and 90°.

11. The device according to one of claims 9 or 10 wherein the gripping end (311) comprises a couple of rings (313) into which the fingers of the operator are inserted.

12. The device according to any one of the preceding claims wherein the dilator body (2) and the introducing body (3) are made of transparent polymeric material.

13. The device according to claim 1 wherein the gripping element (23) has a hooking site (26) that is couplable with a sucking device.
